# EUROPEAN PATENT SPECIFICATION

(11) **EP 3 024 513 B1**
(45) Date of publication and mention of the grant of the patent: **09.05.2018**
(21) Application number: 14744724.7
(22) Date of filing: 10.07.2014
(51) Int. Cl.: A61M 5/00, A61M 5/145, A61B 5/055, A61B 90/00, G01R 33/28, G01R 33/36

(54) **INJECTOR WITH PIEZO MOTOR SYSTEM WITH WAVE FORM TUNING**
INJEKTOR MIT EINEM PIEZOMOTORSYSTEM MIT WELLENFORMABSTIMMUNG
INJECTEUR POURVU D'UN SYSTÈME DE MOTEUR PIÉZOÉLECTRIQUE AVEC RÉGLAGE DE FORME D'ONDE

(30) Priority: 25.07.2013 US 201361858189 P
(43) Date of publication of application: 01.06.2016
(73) Proprietor: Liebel-Flarsheim Company LLC, Cincinnati, OH 45237 (US)
(72) Inventor: CRAWFORD, Charles H., Batavia, OH 45103 (US); RIGGLE, Jeremy A., Cincinnati, OH 45216 (US)
(74) Representative: Edson, Russell Gregory
(86) International application number: PCT/US2014/046180
(87) International publication number: WO 2015/013046

(56) References cited:
- WO-A1-2011/046857
- WO-A2-02/082113
- WO-A2-2007/134146

## Description

### FIELD OF THE INVENTION

The present invention generally relates to magnetic resonance imaging and, more particularly, to motors that are used by one or more components that are utilized in relation to magnetic resonance imaging.

### BACKGROUND

Various medical procedures require that one or more medical fluids be injected into a patient. For example, medical imaging procedures often times Involve the injection of contrast media into a patient, possibly along with saline and/or other fluids. Other medical procedures involve injecting one or more fluids into a patient for therapeutic purposes. Power injectors may be used for these types of applications.

A power injector generally includes what is commonly referred to as a powerhead. One or more syringes may be mounted to the powerhead in various manners (e.g., detachably; rear-loading; front-loading; side-loading). Each syringe typically includes what may be characterized as a syringe plunger, piston, or the like. Each such syringe plunger is designed to interface with (e.g., contact and/or temporarily Interconnect with) an appropriate syringe plunger driver that is incorporated into the powerhead, such that operation of the syringe plunger driver axially advances the associated syringe plunger inside and relative to a barrel of the syringe.

A motor is typically utilized by the power injector to move the syringe plunger driver. As such, the motor is required to operate at an operational frequency range that is specified for the power injector. Currently, piezo motors in general are tuned by annealing the ceramic material of the motor stators. Tuning piezo motors by annealing may add cost and time to the manufacturing of the piezo motors, and may result in limited applications of the motor. For example, once a motor has been tuned by annealing, the piezo motor can only be used in applications requiring an operational frequency range within the frequency range that the motor has been tuned to. As such, motors tuned by annealing are typically tuned to a broad frequency range, which may result in the motor heating more often and ultimately increasing the potential for motor errors. For example, as the motor is operated the motor may heat up, and the more the motor heats up, the more the resonant frequency of the motor may change, which may result in more or less torque than desired. Piezo motors that have been tuned by annealing have been used in power injectors, although the tuned frequency range of these piezo motors is significantly larger than the operational frequency range that is specified for the power injector. For instance and in one known example, a piezo motor may be tuned to a frequency range of 35 KHz - 49 KHz for use in a power injector that requires an operational frequency range of 42.7 KHz - 44.6 KHz. In other words, the piezo motor in this example is tuned to operate at a broad range of frequencies rather than the specific operational frequency range that is actually required by the power injector.

WO 2011/046857 A1 is directed to a syringe plunger driver assembly for advancing a syringe plunger in at least one direction, the assembly may be constructed of all non-ferrous components and includes a piezoelectric pump.

### SUMMARY

A first aspect of the present invention is directed to a power injector. This power injector includes a syringe plunger driver, a piezo motor having a tuned frequency range, and a control board that is operatively interconnected to the piezo motor. A lowest frequency of the tuned frequency range for the piezo motor is no more than 4 KHz from a lowest frequency of an operational frequency range of the power injector. A highest frequency of the tuned frequency range for the piezo motor is no more than 4 KHz from a highest frequency of the operational frequency range of the power injector. The piezo motor is operatively interconnected to the syringe plunger driver for moving the syringe plunger driver in at least a first direction. The control board is operatively interconnected to the piezo motor for applying at least one signal of a selected frequency to the piezo motor that is within the operational frequency range for the power injector.

A number of feature refinements and additional features are applicable to the first aspect of the present invention. These feature refinements and additional features may be used individually or in any combination. As such, each of the following features that will be discussed may be, but are not required to be, used with any other feature or combination of features of the first aspect. The following discussion is applicable to the first aspect, up to the start of the discussion of a second aspect of the present invention.

The power injector may further include a syringe, which may include a syringe plunger (e.g., movable within and relative to a syringe barrel). The syringe plunger driver may interact with the syringe plunger in any appropriate manner to move the syringe plunger when the syringe plunger driver is moved by the piezo motor. The syringe plunger driver may include a ram that is mounted on and movable along a threaded drive screw. In any case, the piezo motor may move the syringe plunger driver (and thereby the syringe plunger) in at least a first direction (e.g., a fluid discharge direction, for instance along an axial path). The piezo motor may move the syringe plunger driver in both a fluid discharge direction and in a retraction direction (e.g., along a common axial path, but in opposite directions).

At least part of the piezo motor may be fabricated from Copper Alloy No. 642. In one embodiment, each stator and each rotor of the piezo motor is formed from Copper Alloy No. 642. The entire piezo motor may be of a configuration that includes no lead, no piezoelectric crystals, or both no lead and no piezoelectric crystals.

The specific frequency range that the piezo motor is tuned to may be selected based on at least one requirement of the application for the piezo motor, which in the case of the first aspect is as a power source for a power injector. In this example, the power injector may have a control board with software requiring an operational frequency range for the power injector. As such and in this example, the piezo motor may be tuned to a specific frequency range that is similar to that of the required operational frequency range of the power injector. In turn, the piezo motor may operate at the required operational frequency range of the power injector. As can be appreciated, the same piezo motor may be tuned to different specific frequency ranges based on different and any number of requirements. As such, the same piezo motor could be tuned for use with any application and/or component requiring the use of a piezo motor including, but not limited to, other magnetic resonance imaging systems.

The specific frequency range that the piezo motor is tuned to may be substantially similar to (e.g., have a small deviation from) the required operational frequency range for the relevant application. For example and in the case of a power injector for the first aspect, a lowest frequency of the specific frequency range for the piezo motor may be no more than 1 KHz from the lowest frequency of the operational frequency range of the power injector, and a highest frequency of the specific frequency range for the piezo motor may be no more than 1 KHz from the highest frequency of the operational frequency range of the power injector. In another example, a lowest frequency of the specific frequency range for the piezo motor may be no more than 2 KHz from the lowest frequency of the operational frequency range of the power injector, and a highest frequency of the specific frequency range for the piezo motor may be no more than 2 KHz from the highest frequency of the operational frequency range of the power injector. In yet another example, a lowest frequency of the specific frequency range for the piezo motor may be no more than 3 KHz from the lowest frequency of the operational frequency range of the power injector, and a highest frequency of the specific frequency range for the piezo motor may be no more than 3 KHz from the highest frequency of the operational frequency range of the power injector. In one embodiment, the operational frequency range for the power injector is a subset of the tuned frequency range for the piezo motor. Another characterization is that the lowest frequency of the specific frequency range for the piezo motor is smaller than the lowest frequency of the operational frequency range of the power injector, and a highest frequency of the specific frequency range for the piezo motor is higher than the highest frequency of the operational frequency range of the power injector.

A second aspect of the present invention is directed to integrating a piezo motor with a magnetic resonance imaging system component. Integrating the piezo motor with the magnetic resonance imaging system component generally includes tuning the piezo motor to a specific frequency range, along with incorporating the tuned piezo motor into a magnetic resonance imaging system component. Tuning the piezo motor to a specific frequency range includes executing a first applying step, which includes applying an initial pressure to the piezo motor. Tuning the piezo motor to a specific frequency range further includes a control board generating at least one signal having a selected frequency that is within the specific frequency range (e.g., the frequency range to which it is desired to tune the piezo motor), executing a second applying step in the form of applying the generated signal to the piezo motor, measuring a vibration frequency of the piezo motor (e.g., the frequency at which the motor resonates/vibrates), and varying the applied pressured to the piezo motor. The second applying step, the measuring step, and the varying step may be repeated until the measured vibration frequency is at a resonant frequency that encompasses the specific frequency range (e.g., such that the piezo motor resonates at/over the specific frequency range).

A number of feature refinements and additional features are applicable to the second aspect of the present invention. These feature refinements and additional features may be used individually or in any combination. As such, each of the following features that will be discussed may be, but are not required to be, used with any other feature or combination of features of the second aspect. The following discussion is applicable to at least the second aspect.

Tuning the piezo motor to a specific frequency range may include a number of steps. For example, at least one signal may be generated via a control board, where this signal has a selected frequency within the specific frequency range (e.g., which includes an operational frequency range of the magnetic resonance imaging system component). In one example, the selected frequency may be a center frequency of the operational frequency range of the magnetic resonance imaging system component that will incorporate the tuned piezo motor (e.g., a power injector). In this example, the piezo motor is tuned to the center frequency of the operational frequency range for the magnetic resonance imaging system component such that the piezo motor resonates at the operational frequency range (e.g., the resonant frequency of the piezo motor has a bandwidth at least equivalent to the operational frequency range of the magnetic resonance imaging system component).

The piezo motor may include a first stator, a second stator, a first rotor and a second rotor. Another step for tuning the piezo motor may include executing a second applying step, which may include applying the generated signal to the piezo motor. The second applying step may also include applying the generated signal to one of the first stator or the second stator. The generated signal may include a plurality of pulses, where each pulse has a pulse width. The generated signal may have a duty cycle in the range from about 15% to about 40%. For example, in one instance of applying the generated signal to the piezo motor, it may be beneficial to have a duty cycle of 15% and in another instance of applying the generated signal to the piezo motor, it may be beneficial to have a duty cycle of 35%. As such, the control board may be operable to adjust the pulse width of each of the plurality of pulses.

Tuning the piezo motor to a specific frequency range may further include measuring a vibration frequency of the piezo motor (e.g., the frequency at which the motor resonates/vibrates) and varying the applied pressured to the piezo motor. Varying the applied pressure to the piezo motor may generally include changing an amount of compression between the first and second stators. For example, varying the applied pressure may include adding at least one shim between the first stator and the second stator. In another example, varying the applied pressure may include removing at least one shim from between the first stator and the second stator. In yet another example, varying the applied pressure may include externally compressing opposing ends of a housing portion of the piezo motor.

Tuning the piezo motor to a specific frequency range may further include repeating: applying the generated signal to the piezo motor, measuring the vibration frequency of the piezo motor and varying the applied pressured to the piezo motor until the measured vibration frequency is at a resonant frequency (e.g., such that the piezo motor resonates at the specific frequency range). The control board may be operable to adjust the selected frequency within the specific frequency range. For example, after it is determined that the measured vibration frequency is at a resonant frequency, a new signal may be generated with a different selected frequency within the specific frequency range and then this signal may be applied to the piezo motor to verify that the piezo motor operates at the new selected frequency (e.g., the vibration frequency is still at a resonant frequency).

In another embodiment, tuning the piezo motor to a specific frequency range may include generating at least four signals via the control board, where these signals have a selected frequency within the specific frequency range. The selected frequency of each signal of the at least four signals may be the same. The remaining steps for tuning the piezo motor may be similar to the steps discussed above with regard to generating at least one signal. For example, a further step in tuning the piezo motor may include applying the at least four signals to the piezo motor. This may include applying at least two of the four signals to the first stator and at least two of the four signals to the second stator. Each of the four signals may have a phase relationship of 90°. For example, the four signals may be sine, -sine, cosine, and -cosine. Again, similar to generating at least one signal, each of the four signals may include a plurality of pulses, where each pulse has a pulse width. The generated signal may have a duty cycle in the range from about 15% to about 40%. As such, the control board may be operable to adjust the pulse width of each of the plurality of pulses of the four signals.

The present invention may be characterized as more generally pertaining to tuning a piezo motor-tuning the piezo motor to a desired frequency range. As such, the tuning method as described herein may be adapted to tune the piezo motor to any operational frequency for any component/device (e.g., for a target application of any type), and such is within the scope of the present invention. A component or device that includes such a tuned piezo motor is also within the scope of the present invention. In this regard, such a tuned motor may be used in magnetic resonance imaging applications and as described, for instance by a power injector or any other magnetic resonance imaging system component. Specifically, piezo motors may be used by one or more components that are utilized in relation to magnetic resonance imaging, and the present invention may tune the piezo motors to operate at a specific frequency range based on an operational frequency range of one or more components that are utilized in relation to magnetic resonance imaging. However, a piezo motor that is tuned in accordance with the present invention may be used for any appropriate application, including for non-MRI applications, as exemplified more fully herein.

It can be appreciated that the specific frequency range that the piezo motor is tuned to (e.g., the tuned frequency range of the piezo motor) may be application/component specific and may be different for each and/or any application/component or may the same for each and/or any application/component. For example, a first application/component may have an operational frequency range of from 42 KHz - 44 KHz. In this example, the piezo motor may be tuned to a specific frequency range of from 40 KHz - 46 KHz. Alternatively and in this same example, the piezo motor may be tuned to a specific frequency range of from 41 KHz - 46 KHz or from 41 KHz - 45 KHz. In another example, a second application/component may have an operational frequency range of from 50 KHz - 53 KHz. In this example, the piezo motor may be tuned to a specific frequency range of 47 KHz - 54 KHz. In this regard, the specific frequency range (e.g., the tuned frequency range) for the piezo motor may have a bandwidth in the range of from about 1 KHz to about 10 KHz. In other words, the resonant frequency of the piezo motor may have a bandwidth in the range of from about 1 KHz to about 10 KHz depending on the operational frequency requirements of the magnetic resonance imaging system component. Specifically, the resonant frequency of the piezo motor may have a bandwidth of about 1 KHz, or about 2 KHz, or about 3 KHz, or about 4 KHz, etc., up to about 10 KHz.

A number of feature refinements and additional features are separately applicable to each aspect of the present invention. These feature refinements and additional features may be used individually or in any combination in relation to each of the above-noted aspects. Any "instructions" or logic that may be utilized by any of the various aspects of the present invention may be implemented in any appropriate manner, including without limitation in any appropriate software, firmware, or hardware, using one or more platforms, using one or more processors, using memory of any appropriate type, using any single computer of any appropriate type or a multiple computers of any appropriate type and interconnected in any appropriate manner, or any combination thereof. These instructions or logic may be implemented at any single location or at multiple locations that are interconnected in any appropriate manner (e.g., via any type of network).

Any power injector that may be utilized to provide a fluid discharge may be of any appropriate size, shape, configuration, and/or type. Any such power injector may utilize one or more syringe plunger drivers of any appropriate size, shape, configuration, and/or type, where each such syringe plunger driver is capable of at least bi-directional movement (e.g., a movement in a first direction for discharging fluid; a movement in a second direction for accommodating a loading and/or drawing of fluid and/or so as to return to a position for a subsequent fluid discharge operation), and where each such syringe plunger driver may interact with its corresponding syringe plunger in any appropriate manner (e.g., by mechanical contact; by an appropriate coupling (mechanical or otherwise)) so as to be able to advance the syringe plunger in at least one direction (e.g., to discharge fluid). Each syringe plunger driver may utilize one or more drive sources of any appropriate size, shape, configuration, and/or type. Multiple drive source outputs may be combined in any appropriate manner to advance a single syringe plunger at a given time. One or more drive sources may be dedicated to a single syringe plunger driver, one or more drive sources may be associated with multiple syringe plunger drivers (e.g., incorporating a transmission of sorts to change the output from one syringe plunger to another syringe plunger), or a combination thereof. Representative drive source forms include a brushed or brushless electric motor, a hydraulic motor, a pneumatic motor, a piezoelectric motor, or a stepper motor.

Any such power injector may be used for any appropriate application where the delivery of one or more medical fluids is desired, including without limitation any appropriate medical imaging application (e.g., computed tomography or CT imaging; magnetic resonance imaging or MRI; single photon emission computed tomography or SPECT imaging; positron emission tomography or PET imaging; X-ray imaging; angiographic imaging; optical imaging; ultrasound imaging) and/or any appropriate medical diagnostic and/or therapeutic application (e.g., injection of chemotherapy, pain management, etc.). Any such power injector may be used in conjunction with any component or combination of components, such as an appropriate imaging system (e.g., a CT scanner). For instance, information could be conveyed between any such power injector and one or more other components (e.g., scan delay information, injection start signal, injection rate).

Any appropriate number of syringes may be utilized with any such power injector in any appropriate manner (e.g., detachably; front-loaded; rear-loaded; side-loaded), any appropriate medical fluid may be discharged from a given syringe of any such power injector (e.g., contrast media, therapeutic fluid, a radiopharmaceutical, saline, and any combination thereof), and any appropriate fluid may be discharged from a multiple syringe power injector configuration in any appropriate manner (e.g., sequentially, simultaneously), or any combination thereof. In one embodiment, fluid discharged from a syringe by operation of the power injector is directed into a conduit (e.g., medical tubing set), where this conduit is fluidly interconnected with the syringe in any appropriate manner and directs fluid to a desired location (e.g., to a catheter that is inserted into a patient for injection). Multiple syringes may discharge into a common conduit (e.g., for provision to a single injection site), or one syringe may discharge into one conduit (e.g., for provision to one injection site), while another syringe may discharge into a different conduit (e.g., for provision to a different injection site). In one embodiment, each syringe includes a syringe barrel and a plunger that is disposed within and movable relative to the syringe barrel. This plunger may interface with the power injector's syringe plunger drive assembly such that the syringe plunger drive assembly is able to advance the plunger in at least one direction, and possibly in two different, opposite directions.

As used herein, the term "fluidly interconnected" refers to two or more components or entities being connected (directly or indirectly) in a manner such that fluid can flow (e.g., unidirectionally or bidirectionally) in a predetermined flow path therebetween. For example, "an injection device fluidly interconnected to a patient" describes a configuration where fluid can flow from the injection device through any interconnecting devices (e.g., tubing, connectors) and into the patient (e.g., into the vasculature of the patient).

Any feature of the present invention that is intended to be limited to a "singular" context or the like will be clearly set forth herein by terms such as "only," "single," "limited to," or the like. Merely introducing a feature in accordance with commonly accepted antecedent basis practice does not limit the corresponding feature to the singular (e.g., indicating that a power injector includes "a control board" alone does not mean that the power injector includes only a single control board). Moreover, any failure to use phrases such as "at least one" also does not limit the corresponding feature to the singular (e.g., indicating that a power injector includes "a control board" alone does not mean that the power injector includes only a single control board). Use of the phrase "at least generally" or the like in relation to a particular feature encompasses the corresponding characteristic and insubstantial variations thereof (e.g., indicating that a syringe barrel is at least generally cylindrical encompasses the syringe barrel being cylindrical). Finally, a reference of a feature in conjunction with the phrase "in one embodiment" does not limit the use of the feature to a single embodiment.

### BRIEF DESCRIPTION OF THE FIGURES

Figure 1 is a schematic of one embodiment of a power injector.
Figure 2A is a perspective view of one embodiment of a portable, stand-mounted, dual-head power injector.
Figure 2B is an enlarged, partially exploded, perspective view of a powerhead used by the power injector of Figure 2A.
Figure 2C is a schematic of one embodiment of a syringe plunger drive assembly that may be used by the power injector of Figure 2A.
Figure 3 is a perspective view of one embodiment of a piezo motor that may be used with the power injector of Figure 2A.
Figure 4 is a schematic of one embodiment of a system for tuning a piezo motor.
Figure 5 is one embodiment of a tuning protocol that may be used by the system of Figure 4 for tuning a piezo motor.
Figure 6 is a schematic of one embodiment of a power injector that includes a piezo motor that has been tuned in accordance with Figures 4-5.

### DETAILED DESCRIPTION

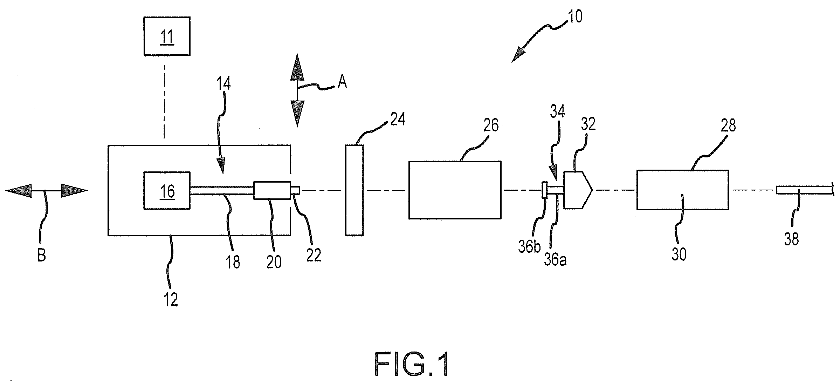

Figure 1 presents a schematic of one embodiment of a power injector 10 having a powerhead 12. One or more graphical user interfaces or GUIs 11 may be associated with the powerhead 12. Each GUI 11: 1) may be of any appropriate size, shape, configuration, and/or type; 2) may be operatively interconnected with the powerhead 12 in any appropriate manner; 3) may be disposed at any appropriate location; 4) may be configured to provide any of the following functions: controlling one or more aspects of the operation of the power injector 10; inputting/editing one or more parameters associated with the operation of the power injector 10; and displaying appropriate information (e.g., associated with the operation of the power injector 10); or 5) any combination of the foregoing. Any appropriate number of GUIs 11 may be utilized. In one embodiment, the power injector 10 includes a GUI 11 that is incorporated by a console that is separate from but which communicates with the powerhead 12. In another embodiment, the power injector 10 includes a GUI 11 that is part of the powerhead 12. In yet another embodiment, the power injector 10 utilizes one GUI 11 on a separate console that communicates with the powerhead 12, and also utilizes another GUI 11 that is on the powerhead 12. Each GUI 11 could provide the same functionality or set of functionalities, or the GUIs 11 may differ in at least some respect in relation to their respective functionalities.

A syringe 28 may be installed on the powerhead 12 and, when installed, may be considered to be part of the power injector 10. Some injection procedures may result in a relatively high pressure being generated within the syringe 28. In this regard, it may be desirable to dispose the syringe 28 within a pressure jacket 26. The pressure jacket 26 is typically associated with the powerhead 12 in a manner that allows the syringe 28 to be disposed therein as a part of or after installing the syringe 28 on the powerhead 12. The same pressure jacket 26 will typically remain associated with the powerhead 12, as various syringes 28 are positioned within and removed from the pressure jacket 26 for multiple injection procedures. The power injector 10 may eliminate the pressure jacket 26 if the power injector 10 is configured/utilized for low-pressure injections and/or if the syringe(s) 28 to be utilized with the power injector 10 is (are) of sufficient durability to withstand high-pressure injections without the additional support provided by a pressure jacket 26. In any case, fluid discharged from the syringe 28 may be directed into a conduit 38 of any appropriate size, shape, configuration, and/or type, which may be fluidly interconnected with the syringe 28 in any appropriate manner, and which may direct fluid to any appropriate location (e.g., to a patient).

The powerhead 12 includes a syringe plunger drive assembly or syringe plunger driver 14 that interacts (e.g., interfaces) with the syringe 28 (e.g., a plunger 32 thereof) to discharge fluid from the syringe 28. This syringe plunger drive assembly 14 includes a drive source 16 (e.g., a motor of any appropriate size, shape, configuration, and/or type, optional gearing, and the like) that powers a drive output 18 (e.g., a rotatable drive screw). A ram 20 may be advanced along an appropriate path (e.g., axial) by the drive output 18. The ram 20 may include a coupler 22 for interacting or interfacing with a corresponding portion of the syringe 28 in a manner that will be discussed below.

The syringe 28 includes a plunger or piston 32 that is movably disposed within a syringe barrel 30 (e.g., for axial reciprocation along an axis coinciding with the double-headed arrow B). The plunger 32 may include a coupler 34. This syringe plunger coupler 34 may interact or interface with the ram coupler 22 to allow the syringe plunger drive assembly 14 to retract the syringe plunger 32 within the syringe barrel 30. The syringe plunger coupler 34 may be in the form of a shaft 36a that extends from a body of the syringe plunger 32, together with a head or button 36b. However, the syringe plunger coupler 34 may be of any appropriate size, shape, configuration, and/or type.

Generally, the syringe plunger drive assembly 14 of the power injector 10 may interact with the syringe plunger 32 of the syringe 28 in any appropriate manner (e.g., by mechanical contact; by an appropriate coupling (mechanical or otherwise)) so as to be able to move or advance the syringe plunger 32 (relative to the syringe barrel 30) in at least one direction (e.g., to discharge fluid from the corresponding syringe 28). That is, although the syringe plunger drive assembly 14 may be capable of bi-directional motion (e.g., via operation of the same drive source 16), the power injector 10 may be configured such that the operation of the syringe plunger drive assembly 14 actually only moves each syringe plunger 32 being used by the power injector 10 in only one direction. However, the syringe plunger drive assembly 14 may be configured to interact with each syringe plunger 32 being used by the power injector 10 so as to be able to move each such syringe plunger 32 in each of two different directions (e.g. in different directions along a common axial path).

Retraction of the syringe plunger 32 may be utilized to accommodate a loading of fluid into the syringe barrel 30 for a subsequent injection or discharge, may be utilized to actually draw fluid into the syringe barrel 30 for a subsequent injection or discharge, or for any other appropriate purpose. Certain configurations may not require that the syringe plunger drive assembly 14 be able to retract the syringe plunger 32, in which case the ram coupler 22 and syringe plunger coupler 34 may not be desired. In this case, the syringe plunger drive assembly 14 may be retracted for purposes of executing another fluid delivery operation (e.g., after another pre-filled syringe 28 has been installed). Even when a ram coupler 22 and syringe plunger coupler 34 are utilized, these components may or may not be coupled when the ram 20 advances the syringe plunger 32 to discharge fluid from the syringe 28 (e.g., the ram 20 may simply "push on" the syringe plunger coupler 34 or directly on a proximal end of the syringe plunger 32). Any single motion or combination of motions in any appropriate dimension or combination of dimensions may be utilized to dispose the ram coupler 22 and syringe plunger coupler 34 in a coupled state or condition, to dispose the ram coupler 22 and syringe plunger coupler 34 in an un-coupled state or condition, or both.

The syringe 28 may be installed on the powerhead 12 in any appropriate manner. For instance, the syringe 28 could be configured to be installed directly on the powerhead 12. In the illustrated embodiment, a housing 24 is appropriately mounted on the powerhead 12 to provide an interface between the syringe 28 and the powerhead 12. This housing 24 may be in the form of an adapter to which one or more configurations of syringes 28 may be installed, and where at least one configuration for a syringe 28 could be installed directly on the powerhead 12 without using any such adapter. The housing 24 may also be in the form of a faceplate to which one or more configurations of syringes 28 may be installed. In this case, it may be such that a faceplate is required to install a syringe 28 on the powerhead 12 - the syringe 28 could not be installed on the powerhead 12 without the faceplate. When a pressure jacket 26 is being used, it may be installed on the powerhead 12 in the various manners discussed herein in relation to the syringe 28, and the syringe 28 will then thereafter be installed in the pressure jacket 26.

The housing 24 may be mounted on and remain in a fixed position relative to the powerhead 12 when installing a syringe 28. Another option is to movably interconnect the housing 24 and the powerhead 12 to accommodate installing a syringe 28. For instance, the housing 24 may move within a plane that contains the double-headed arrow A to provide one or more of coupled state or condition and an un-coupled state or condition between the ram coupler 22 and the syringe plunger coupler 34.

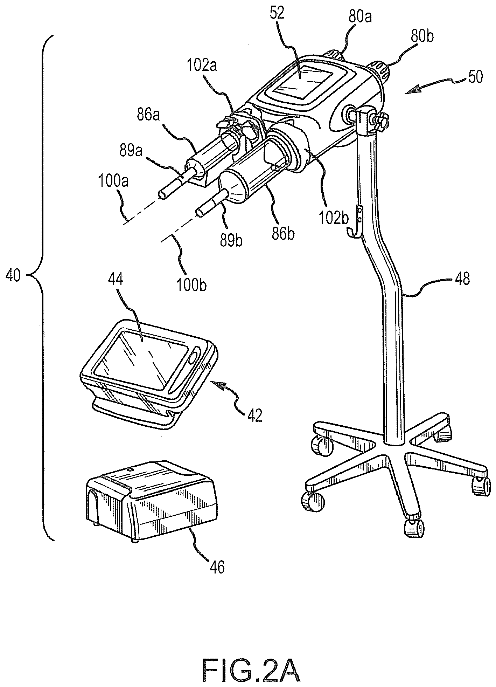

One particular power injector configuration is illustrated in Figure 2A, is identified by a reference numeral 40, and is at least generally in accordance with the power injector 10 of Figure 1. The power injector 40 includes a powerhead 50 that is mounted on a portable stand 48. Two syringes 86a, 86b for the power injector 40 are mounted on the powerhead 50. Fluid may be discharged from the syringes 86a, 86b during operation of the power injector 40.

The portable stand 48 may be of any appropriate size, shape, configuration, and/or type. Wheels, rollers, casters, or the like may be utilized to make the stand 48 portable. The powerhead 50 could be maintained in a fixed position relative to the portable stand 48. However, it may be desirable to allow the position of the powerhead 50 to be adjustable relative to the portable stand 48 in at least some manner. For instance, it may be desirable to have the powerhead 50 in one position relative to the portable stand 48 when loading fluid into one or more of the syringes 86a, 86b, and to have the powerhead 50 in a different position relative to the portable stand 48 for performance of an injection procedure. In this regard, the powerhead 50 may be movably interconnected with the portable stand 48 in any appropriate manner (e.g., such that the powerhead 50 may be pivoted through at least a certain range of motion, and thereafter maintained in the desired position).

It should be appreciated that the powerhead 50 could be supported in any appropriate manner for providing fluid. For instance, instead of being mounted on a portable structure, the powerhead 50 could be interconnected with a support assembly, that in turn is mounted to an appropriate structure (e.g., ceiling, wall, floor). Any support assembly for the powerhead 50 may be positionally adjustable in at least some respect (e.g., by having one or more support sections that may be repositioned relative to one or more other support sections), or may be maintained in a fixed position. Moreover, the powerhead 50 may be integrated with any such support assembly so as to either be maintained in a fixed position or so as to be adjustable relative to the support assembly.

The powerhead 50 includes a graphical user interface or GUI 52. This GUI 52 may be configured to provide one or any combination of the following functions: controlling one or more aspects of the operation of the power injector 40; inputting/editing one or more parameters associated with the operation of the power injector 40; and displaying appropriate information (e.g., associated with the operation of the power injector 40). The power injector 40 may also include a console 42 and powerpack 46 that each may be in communication with the powerhead 50 in any appropriate manner (e.g., via one or more cables), that may be placed on a table or mounted on an electronics rack in an examination room or at any other appropriate location, or both. The powerpack 46 may include one or more of the following and in any appropriate combination: a power supply for the injector 40; interface circuitry for providing communication between the console 42 and powerhead 50; circuitry for permitting connection of the power injector 40 to remote units such as remote consoles, remote hand or foot control switches, or other original equipment manufacturer (OEM) remote control connections (e.g., to allow for the operation of power injector 40 to be synchronized with the x-ray exposure of an imaging system); and any other appropriate componentry. The console 42 may include a touch screen display 44, which in turn may provide one or more of the following functions and in any appropriate combination: allowing an operator to remotely control one or more aspects of the operation of the power injector 40; allowing an operator to enter/edit one or more parameters associated with the operation of the power injector 40; allowing an operator to specify and store programs for automated operation of the power injector 40 (which can later be automatically executed by the power injector 40 upon initiation by the operator); and displaying any appropriate information in relation to the power injector 40 and including any aspect of its operation.

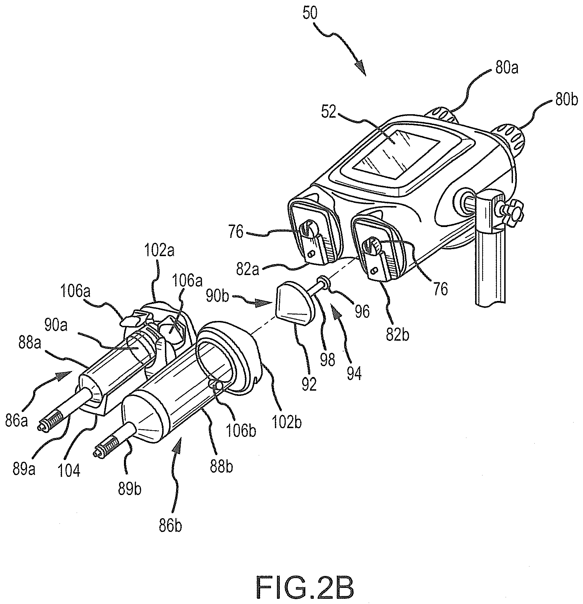

Various details regarding the integration of the syringes 86a, 86b with the powerhead 50 are presented in Figure 2B. Each of the syringes 86a, 86b includes the same general components. The syringe 86a includes a plunger or piston 90a that is movably disposed within a syringe barrel 88a. Movement of the plunger 90a along an axis 100a (Figure 2A) via operation of the powerhead 50 will discharge fluid from within a syringe barrel 88a through a nozzle 89a of the syringe 86a. An appropriate conduit (not shown) will typically be fluidly interconnected with the nozzle 89a in any appropriate manner to direct fluid to a desired location (e.g., a patient). Similarly, the syringe 86b includes a plunger or piston 90b that is movably disposed within a syringe barrel 88b. Movement of the plunger 90b along an axis 100b (Figure 2A) via operation of the powerhead 50 will discharge fluid from within the syringe barrel 88b through a nozzle 89b of the syringe 86b. An appropriate conduit (not shown) will typically be fluidly interconnected with the nozzle 89b in any appropriate manner to direct fluid to a desired location (e.g., a patient).

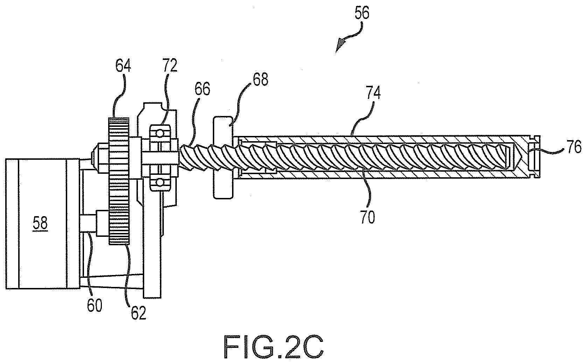

The syringe 86a is interconnected with the powerhead 50 via an intermediate faceplate 102a. This faceplate 102a includes a cradle 104 that supports at least part of the syringe barrel 88a, and which may provide/accommodate any additional functionality or combination of functionalities. A mounting 82a is disposed on and is fixed relative to the powerhead 50 for interfacing with the faceplate 102a. A ram coupler 76 of a ram 74 (Figure 2C), which are each part of a syringe plunger drive assembly or syringe plunger driver 56 (Figure 2C) for the syringe 86a, is positioned in proximity to the faceplate 102a when mounted on the powerhead 50. Details regarding the syringe plunger drive assembly 56 will be discussed in more detail below in relation to Figure 2C. Generally, the ram coupler 76 may be coupled with the syringe plunger 90a of the syringe 86a, and the ram coupler 76 and ram 74 (Figure 2C) may then be moved relative to the powerhead 50 to move the syringe plunger 90a along the axis 100a (Figure 2A). It may be such that the ram coupler 76 is engaged with, but not actually coupled to, the syringe plunger 90a when moving the syringe plunger 90a to discharge fluid through the nozzle 89a of the syringe 86a.

The faceplate 102a may be moved at least generally within a plane that is orthogonal to the axes 100a, 100b (associated with movement of the syringe plungers 90a, 90b, respectively, and illustrated in Figure 2A), both to mount the faceplate 102a on and remove the faceplate 102a from its mounting 82a on the powerhead 50. The faceplate 102a may be used to couple the syringe plunger 90a with its corresponding ram coupler 76 on the powerhead 50. In this regard, the faceplate 102a includes a pair of handles 106a. Generally and with the syringe 86a being initially positioned within the faceplate 102a, the handles 106a may be moved to in turn move/translate the syringe 86a at least generally within a plane that is orthogonal to the axes 100a, 100b (associated with movement of the syringe plungers 90a, 90b, respectively, and illustrated in Figure 2A). Moving the handles 106a to one position moves/translates the syringe 86a (relative to the faceplate 102a) in an at least generally downward direction to couple its syringe plunger 90a with its corresponding ram coupler 76. Moving the handles 106a to another position moves/translates the syringe 86a (relative to the faceplate 102a) in an at least generally upward direction to uncouple its syringe plunger 90a from its corresponding ram coupler 76.

The syringe 86b is interconnected with the powerhead 50 via an intermediate faceplate 102b. A mounting 82b is disposed on and is fixed relative to the powerhead 50 for interfacing with the faceplate 102b. A ram coupler 76 of a ram 74 (Figure 2C), which are each part of a syringe plunger drive assembly 56 for the syringe 86b, is positioned in proximity to the faceplate 102b when mounted to the powerhead 50. Details regarding the syringe plunger drive assembly 56 again will be discussed in more detail below in relation to Figure 2C. Generally, the ram coupler 76 may be coupled with the syringe plunger 90b of the syringe 86b, and the ram coupler 76 and ram 74 (Figure 2C) may be moved relative to the powerhead 50 to move the syringe plunger 90b along the axis 100b (Figure 2A). It may be such that the ram coupler 76 is engaged with, but not actually coupled to, the syringe plunger 90b when moving the syringe plunger 90b to discharge fluid through the nozzle 89b of the syringe 86b.

The faceplate 102b may be moved at least generally within a plane that is orthogonal to the axes 100a, 100b (associated with movement of the syringe plungers 90a, 90b, respectively, and illustrated in Figure 2A), both to mount the faceplate 102b on and remove the faceplate 102b from its mounting 82b on the powerhead 50. The faceplate 102b also may be used to couple the syringe plunger 90b with its corresponding ram coupler 76 on the powerhead 50. In this regard, the faceplate 102b may include a handle 106b. Generally and with the syringe 86b being initially positioned within the faceplate 102b, the syringe 86b may be rotated along its long axis 100b (Figure 2A) and relative to the faceplate 102b. This rotation may be realized by moving the handle 106b, by grasping and turning the syringe 86b, or both. In any case, this rotation moves/translates both the syringe 86b and the faceplate 102b at least generally within a plane that is orthogonal to the axes 100a, 100b (associated with movement of the syringe plungers 90a, 90b, respectively, and illustrated in Figure 2A). Rotating the syringe 86b in one direction moves/translates the syringe 86b and faceplate 102b in an at least generally downward direction to couple the syringe plunger 90b with its corresponding ram coupler 76. Rotating the syringe 86b in the opposite direction moves/translates the syringe 86b and faceplate 102b in an at least generally upward direction to uncouple its syringe plunger 90b from its corresponding ram coupler 76.

As illustrated in Figure 2B, the syringe plunger 90b includes a plunger body 92 and a syringe plunger coupler 94. This syringe plunger coupler 94 includes a shaft 98 that extends from the plunger body 92, along with a head 96 that is spaced from the plunger body 92. Each of the ram couplers 76 includes a larger slot that is positioned behind a smaller slot on the face of the ram coupler 76. The head 96 of the syringe plunger coupler 94 may be positioned within the larger slot of the ram coupler 76, and the shaft 98 of the syringe plunger coupler 94 may extend through the smaller slot on the face of the ram coupler 76 when the syringe plunger 90b and its corresponding ram coupler 76 are in a coupled state or condition. The syringe plunger 90a may include a similar syringe plunger coupler 94 for interfacing with its corresponding ram coupler 76.

The powerhead 50 is utilized to discharge fluid from the syringes 86a, 86b in the case of the power injector 40. That is, the powerhead 50 provides the motive force to discharge fluid from each of the syringes 86a, 86b. One embodiment of what may be characterized as a syringe plunger drive assembly or syringe plunger driver is illustrated in Figure 2C, is identified by reference numeral 56, and may be utilized by the powerhead 50 to discharge fluid from each of the syringes 86a, 86b. A separate syringe plunger drive assembly 56 may be incorporated into the powerhead 50 for each of the syringes 86a, 86b. In this regard and referring back to Figures 2A-B, the powerhead 50 may include hand-operated knobs 80a and 80b for use in separately controlling each of the syringe plunger drive assemblies 56.

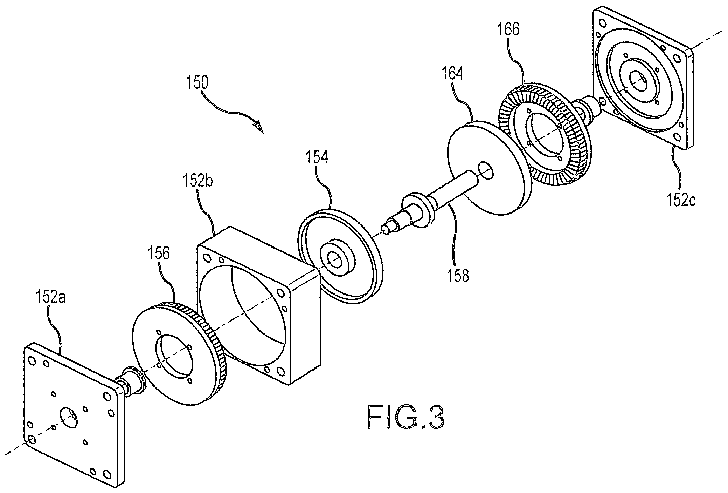

Initially and in relation to the syringe plunger drive assembly 56 of Figure 2C, each of its individual components may be of any appropriate size, shape, configuration and/or type. The syringe plunger drive assembly 56 includes a motor 58, which has an output shaft 60. Details regarding one embodiment for the motor 58 will be discussed in more detail below in relation to Figure 3. A drive gear 62 is mounted on and rotates with the output shaft 60 of the motor 58. The drive gear 62 is engaged or is at least engageable with a driven gear 64. This driven gear 64 is mounted on and rotates with a drive screw or shaft 66. One or more bearings 72 appropriately support the drive screw 66. A nut 68 is provided for engaging the driver screw 66.

A carriage or ram 74 is movably mounted on the drive screw 66, and extends from the nut 68 such that the nut 68 and ram 74 move collectively. Generally, rotation of the drive screw 66 in one direction axially advances the ram 74 along the drive screw 66 in the direction of the corresponding syringe 86a/b, while rotation of the drive screw 66 in the opposite direction axially advances the ram 74 along the drive screw 66 away from the corresponding syringe 86a/b. In this regard, the perimeter of at least part of the drive screw 66 includes helical threads 70 that interface with at least part of the ram 74 (more specifically, the nut 68). The rotation of the drive screw 66 provides for an axial movement of the ram 74 in a direction determined by the rotational direction of the drive screw 66. The ram 74 includes a coupler 76 that that may be detachably coupled with a syringe plunger coupler 94 of the syringe plunger 90a/b of the corresponding syringe 86a/b. When the ram coupler 76 and syringe plunger coupler 94 are appropriately coupled, the syringe plunger 90a/b moves along with ram 74.

The power injectors 10, 40 of Figures 1 and 2A-C each may be used for any appropriate application, including without limitation for medical imaging applications where fluid is injected into a subject (e.g., a patient) and/or any appropriate medical diagnostic and/or therapeutic application (e.g., injection of chemotherapy, pain management, etc.). Representative medical imaging applications for the power injectors 10, 40 include without limitation computed tomography or CT imaging, magnetic resonance imaging or MRI, single photon emission computed tomography or SPECT imaging, positron emission tomography or PET imaging, X-ray imaging, angiographic imaging, optical imaging, and ultrasound imaging. The power injectors 10, 40 each could be used alone or in combination with one or more other components. The power injectors 10, 40 each may be operatively interconnected with one or more components, for instance so that information may be conveyed between the power injector 10, 40 and one or more other components (e.g., scan delay information, injection start signal, injection rate).

Any number of syringes may be utilized by each of the power injectors 10, 40, including without limitation single-head configurations (for a single syringe) and dual-head configurations (for two syringes). In the case of a multiple syringe configuration, each power injector 10, 40 may discharge fluid from the various syringes in any appropriate manner and according to any timing sequence (e.g., sequential discharges from two or more syringes, simultaneous discharges from two or more syringes, or any combination thereof). Multiple syringes may discharge into a common conduit (e.g., for provision to a single injection site), or one syringe may discharge into one conduit (e.g., for provision to one injection site), while another syringe may discharge into a different conduit (e.g., for provision to a different injection site). Each such syringe utilized by each of the power injectors 10, 40 may include any appropriate fluid (e.g., a medical fluid), for instance contrast media, therapeutic fluid, a radiopharmaceutical, saline, and any combination thereof. Each such syringe utilized by each of the power injectors 10, 40 may be installed in any appropriate manner (e.g., rear-loading configurations may be utilized; front-loading configurations may be utilized; side-loading configurations may be utilized).

Figure 3 illustrates one embodiment of a piezo motor 150 that may be used by a power injector and that is particularly suited for magnetic resonance imaging applications. However, the piezo motor 150 may be incorporated by other components that are used in magnetic resonance imaging ("magnetic resonance imaging system components"). Hereafter, the piezo motor 150 may be described when used in place of the motor 58 for the power injector 40 of Figures 2A-2C, although it may be used in other power injector configurations, as well as by other magnetic resonance imaging system components. Moreover, the manner of tuning the piezo motor 150 may be applicable to piezo motor applications other than magnetic resonance imaging.

The piezo motor 150 may include housing portions 152a, 152b, 152c, a first rotor 154, a first stator 156, a center shaft 158, a second rotor 164, and a second stator 166. When used for magnetic resonance imaging applications, the entirety of the piezo motor 150 may be devoid of any lead, the entirety of the piezo motor 150 may be devoid of piezoelectric crystals, or both. In one embodiment, at least part of the piezo motor 150 is composed of Copper Alloy No. 642. For example, the first and second stators 156, 166 and first and second rotors 154, 164 each may be composed of Copper Alloy No. 642.

The piezo motor 150 may be operatively interconnected to the syringe plunger driver 56 for driving the syringe plunger driver 56 in at least a first direction. The piezo motor 150 may be tuned to resonate at a frequency range of a bandwidth of less than or equal to 5 KHz (a method for tuning the piezo motor 150 will be described in more detail below). As such, the first and second stators 156, 166 may be configured to operate at specific and narrow operating frequency ranges such that the piezo motor 150 may be utilized for specific applications. For example and in one application, the piezo motor 150 may be used in an MRI suite with a power injector (e.g., power injectors 10, 40) that specifies an operational frequency range of 42.7 KHz - 44.6 KHz. In this application, the piezo motor 150 may be tuned to operate at a frequency range of about 40 KHz - 45 KHz, for example. In another application, the piezo motor 150 may be used in an MRI suite with a robotic arm that specifies an operational frequency range outside the range of 40 KHz - 45 KHz. For this application, the same piezo motor 150 that was used for the power injector application may be tuned to operate at the new operational frequency range. The piezo motor 150 may be tuned to operate at a frequency range as low as 1 KHz (e.g., the resonant frequency of the piezo motor 150 may have a bandwidth of no more than 1 KHz). In other embodiments, the piezo motor 150 may be tuned to operate at a frequency range not greater than 10 KHz. As such, the piezo motor 150 may be tuned for various applications having different and specific operational frequency ranges including, but not limited to, frequency ranges outside of a frequency range that the piezo motor 150 was previously tuned to.

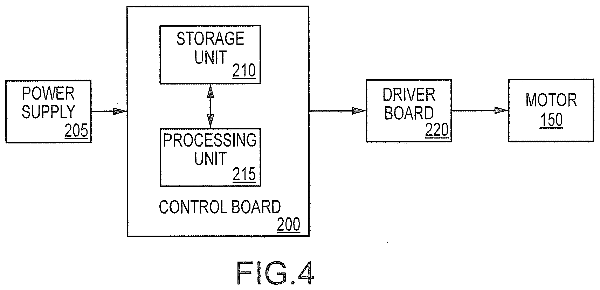

Figure 4 is a schematic of one embodiment of a system for tuning the piezo motor 150. The system of Figure 4 may include a power supply 205, a control board 200, and a driver unit or board 220. The control board 200 may include a storage unit 210 and processing unit 215. In one example, the control board 200 may be configured to tune the piezo motor 150. For example, the control board 200 may be configured to apply at least one signal at a selected frequency to the piezo motor 150, where this selected frequency is within an operational frequency range for the target application (e.g., the operational frequency range required by the power injectors 10, 40). At least one shim (not illustrated) may be used between rotors 154, 164 (Figure 3) to vary the pressure applied against the stators 156, 166 (Figure 3) until the piezo motor 150 resonates within the operational frequency range required by a power injector application (e.g., required by the power injectors 10, 40). In other words, the piezo motor 150 is tuned such that it resonates within the operational frequency range of the device that will incorporate the piezo motor 150. The piezo motor 150 may be tuned to a center frequency of the operational frequency range of the device that will incorporate the piezo motor 150 such that the piezo motor 150 may operate at the lower and higher frequencies of the operational frequency range of the device that will incorporate the piezo motor 150. For example, it may be desirable to adjust the drive frequency to achieve more or less torque of the piezo motor 150 (e.g., to achieve a higher or lower fluid flow rate for a power injector application). In another example, the control board 200 may be configured to apply at least two signals at the selected frequency to the first stator 156 and at least two signals at the selected frequency to the second stator 166. The two signals applied to the first and second stators 156, 166 may have a phase relationship of 90° (e.g., a sine and cosine signal may be applied). Each signal applied to the piezo motor 150 may have a duty cycle of at least about 15% and not more than about 40%. In turn, motor errors and an undesired level of heating may be reduced. For example, applying a signal to the piezo motor 150 having a duty cycle of greater than 40% may cause the piezo motor 150 to heat up more than desired and/or at a faster rate than desired, which may result in the piezo motor 150 producing more or less torque than desired.

As discussed above, each of the power injectors 10, 40 may include at least either a single-head configuration (for a single syringe) or a dual-head configuration (for two syringes). In this regard, in the dual-head configuration, it may be desired to tune two motors. As such, the control board 200 may be configured to select a motor for tuning. As discussed above, it may be desirable to adjust the drive frequency of the signal applied to the piezo motor 150. In this regard, the control board 200 may be configured to adjust the frequency and/or pulse width of each signal applied to the piezo motor 150. In turn, the control board 200 may be configured to select the operating speed of the piezo motor 150. As discussed above in relation to Figure 2C, rotation of the drive screw 66 in one direction axially advances the ram 74 along the drive screw 66 in the direction of the corresponding syringe 86a/b, while rotation of the drive screw 66 in the opposite direction axially advances the ram 74 along the drive screw 66 away from the corresponding syringe 86a/b. As such, the control board 200 may be configured to select the operational direction of the piezo motor 150 (e.g., the direction of rotation of the drive screw 66).

The power supply 205 may be operatively interconnected with the control board 200. For example, the power supply 205 may supply power to the storage unit 210 and the processing unit 215. The storage unit 210 may be memory of the control board 200. For example, the storage unit 210 may store information on a temporary or permanent basis. The storage unit 210 may include read-only memory (ROM) (e.g., PROM, EPROM, EEPROM, EAROM, Flash memory) or read-write memory (e.g., random access memory, hard disk drive, solid state drive), to name a few. The storage unit 210 may be in communication with the processing unit 215 (e.g., one or more processors). The storage unit 210 may send data/information to the processing unit 215 for processing. In this regard, the processing unit 215 may include instructions (e.g., computer code) for processing data/information.

In one example, the processing unit 215 is in the form of a central processing unit (CPU) (e.g., one or more processors disposed in any appropriate processing architecture). The processing unit 215 may include instructions of a computer program, for example, for performing arithmetical, logical, and/or input/output operations of the control board 200. For example, the processing unit 215 may include instructions for generating and applying at least one signal having a selected frequency and pulse width to at least one motor and adjusting the selected frequency and pulse width of the at least one signal. In other words, the processing unit 215 of the control board 200 may perform all the processing necessary to tune a motor (e.g., the piezo motor 150). The driver board 220 may be in communication with the control board 200 and at least one piezo motor 150. For example, the processing unit 215 of the control board 200 may communicate a signal having a generated frequency and pulse width to the driver board 220. The driver board 220 may include output ports (not illustrated) for driving (e.g., applying the signal) the at least one piezo motor 150 via the received signal. The control board 200 may include one or more storage units 210 and processing units 215, and each of the storage units 210 and processing units 215 may be located at the same location or may be located at different location relative to one another such that a motor is appropriately tuned.

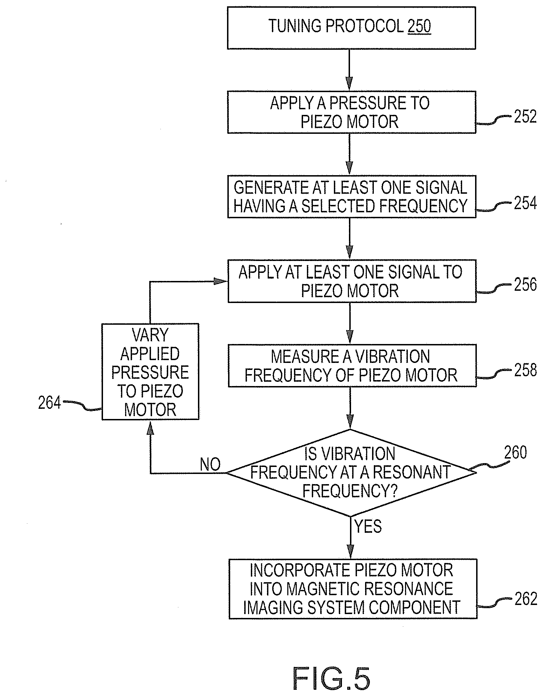

Figure 5 presents an embodiment of a protocol 250 for tuning a piezo motor (e.g., piezo motor 150). Tuning protocol 250 is generally directed to tuning a piezo motor for a specific application such that the motor operates at the operational frequency range required by the specific application. The application may be any application utilized in an MRI suite. For example, one application may include fluid delivery via a power injector (e.g., power injectors 10, 40), as described above. Other applications may include any application requiring the use of a motor and having a required operational frequency range, including for non-MRI applications. Step 252 of tuning protocol 250 is directed to applying a pressure to piezo motor 150. Applying a pressure to the piezo motor 150 may change the resonating frequency of the piezo motor 150 (e.g., the resonating frequency of the first and second stators 156, 166). Applying a pressure to piezo motor 150 may include adding at least one shim between rotors 154, 164 and located on the center shaft 158 to vary the gap between rotors 154, 156. In another example, the pressure may be applied to piezo motor 150 by externally compressing opposing housing portions 152a and 152c of the piezo motor 150.

After an initial pressure is applied to piezo motor 150, the tuning protocol 250 may proceed to step 254. Step 254 is directed to generating at least one signal having a selected frequency (e.g., a driving frequency) within the operational frequency range required by the application for which the piezo motor 150 will be used. In one example, the selected frequency will be the center frequency of the operational frequency range. For example, if the operational frequency range is from about 42.7 KHz - 44.6 KHz, the selected frequency of the signal may be about 43.65 KHz. In some embodiments, step 254 may be directed to generating at least four signals having a selected frequency within the operational frequency range. For example, all four signals may have the same selected frequency and a phase relationship of 90°. For example and in the case where the selected frequency is about 43.65 KHz, all four signals are generated to have a frequency of about 43.65 KHz, but each signal is generated to be 90°out of phase with one another. After at least one signal is generated, the tuning protocol 250 may proceed to step 256. Step 256 is directed to applying the at least one signal to the piezo motor 150. The at least one signal may be applied to one of the first stator 156 or the second stator 166. In the case where four signals are generated, two signals may be applied to the first stator 156 and two signals may be applied to the second stator 166. For example, the two signals applied to the first stator 156 may have the same frequency and are 90° out of phase and the two signals applied to the second stator 166 may have the same frequency and are 90° out of phase.

While the at least one signal is being applied to the piezo motor 150, tuning protocol 250 may proceed to step 258. Step 258 is directed to measuring a vibration frequency of the piezo motor 150. The vibration frequency of the piezo motor 150 is the frequency at which the piezo motor 150 vibrates. After measuring the vibration frequency of the piezo motor 150, it is determined if the vibration frequency is at a resonant frequency of the piezo motor 150. In other words, it is desired for the piezo motor 150 to resonate within the operational frequency range required by the application such that the piezo motor 150 only responds to signals having frequencies within the operational frequency range and such that a specified amount of torque results from each signal having a selected frequency within the operational frequency range. The piezo motor 150 may be tuned to a resonant frequency having a bandwidth greater than that of the operational frequency range. For example, as discussed above, if the operational frequency range is from about 42.7 KHz - 44.6 KHz, the piezo motor 150 may be tuned to operate at a frequency of from about 40 KHz - 45 KHz (e.g., having a resonant frequency bandwidth of 5 KHz).

If the vibration frequency is not at a resonant frequency (step 260), the tuning protocol 250 proceeds to step 264. Step 264 is directed to varying the applied pressure to the piezo motor 150. The pressure may be varied by changing the gap between the first and second rotors 154, 164 by either adding shims to the center shaft 158, taking shims off the center shaft 158, or compressing opposing housing portions 152a and 152c of the piezo motor 150 (as discussed above in relation to step 252 of tuning protocol 250). In other words, varying the pressure may include changing an amount of compression between the first and second rotors 154, 164. After the pressure is varied in step 264, tuning protocol 250 proceeds to and repeats steps 256, 258, 260, and 264 until the measured vibration frequency is at a resonant frequency. In other words, the piezo motor 150 is tuned to resonate within the operational frequency range. If the vibration frequency is at the resonant frequency, the piezo motor 150 is tuned and the tuning protocol 250 proceeds to step 262. Step 262 is directed to incorporating the piezo motor 150 into a magnetic resonance imaging system component.

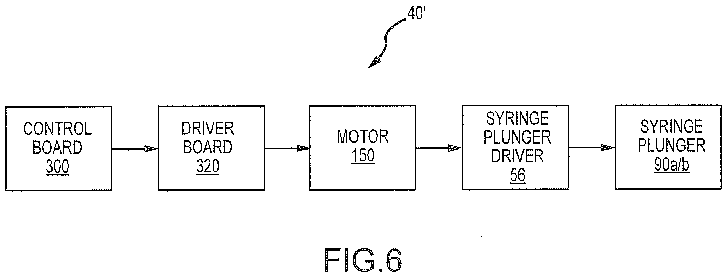

Figure 6 illustrates one embodiment of a power injector 40' that is a variation of the power injector 40 as described above in relation to Figure 2A, and that includes a tuned piezo motor 150. The power injector 40' may include a control board 300, a driver board 320, piezo motor 150 (as described above in relation to Figures 3-5), syringe plunger driver 56 (as described above in relation to Figure 2C), and syringe plunger 90a/b (as described above in relation to Figures 2B-2C). The control board 300 may be configured to operate at the operational frequency range for the power injector 40'. For example, the control board 300 has a set frequency range (e.g., the operational frequency range) at which signals are applied to the piezo motor 150. In other words, the control board 200 discussed above in relation to Figures 4-5 (for tuning the piezo motor 150), may be programmed to functionally mimic control board 300 (used by the power injector 40') when control board 200 is utilized to tune the piezo motor 150. Driver board 320 for power injector 40' may operate the same as driver board 220. In other words, driver board 320 may include output ports (not illustrated) for driving the piezo motor 150 (e.g., applying the signal). As discussed above, the piezo motor 150 may be operatively interconnected to the syringe plunger driver 56 for driving the syringe plunger driver 56 in at least a first direction.

The foregoing description of the present invention has been presented for purposes of illustration and description. Furthermore, the description is not intended to limit the invention to the form disclosed herein. Consequently, variations and modifications commensurate with the above teachings, and skill and knowledge of the relevant art, are within the scope of the present invention. The embodiments described hereinabove are further intended to explain best modes known of practicing the invention and to enable others skilled in the art to utilize the invention in such, or other embodiments and with various modifications required by the particular application(s) or use(s) of the present invention. It is intended that the appended claims be construed to include alternative embodiments to the extent permitted by the prior art.

## Claims

1. A power injector (10) for magnetic resonance imaging, comprising:
a syringe plunger driver (14);
a piezo motor (150) comprising a tuned frequency range, wherein a lowest frequency of said tuned frequency range for said piezo motor (150) is no more than 4 KHz from a lowest frequency of an operational frequency range of said power injector (10), wherein a highest frequency of said tuned frequency range for said piezo motor (150) is no more than 4 KHz from a highest frequency of said operational frequency range of said power injector (10), and wherein said piezo motor (150) is operatively interconnected to said syringe plunger driver (14) for moving said syringe plunger driver (14) in at least a first direction, wherein said operational frequency range of said power injector (10) is a subset of said tuned frequency range of said piezo motor (150); and
a control board (200) operatively interconnected to said piezo motor (150) for applying at least one signal of a selected frequency within said operational frequency range to said piezo motor (150).

2. The power injector (10) of claim 1, further comprising:
a syringe (28) comprising a syringe plunger (32), wherein said syringe plunger driver (14) interacts with said syringe plunger (32) to move said syringe plunger (32) when said syringe plunger driver (14) is moved by said piezo motor (150).

3. The power injector (10) of any of Claims 1-2, wherein said syringe plunger driver (14) comprises a ram (20) that is mounted on and movable along a threaded drive screw (18) and wherein said piezo motor (150) optionally comprises a first stator (156), a second stator (166), a first rotor (154) and a second rotor (164).

4. The power injector (10) of any of Claims 1-3, wherein said lowest frequency of said tuned frequency range for said piezo motor (150) is no more than 3 KHz from said lowest frequency, optionally no more than 2 KHz from said lowest frequency, and optionally no more than 1 KHz from said lowest frequency, of said operational frequency range of said power injector (10); and wherein said highest frequency of said tuned frequency range for said piezo motor (150) is no more than 3 KHz from said highest frequency, optionally no more than 2 KHz from said highest frequency, and optionally no more than 1 KHz from said highest frequency, of said operational frequency range of said power injector (10).

5. The power injector (10) of any of Claims 1-4, wherein said tuned frequency range for said piezo motor (150) has a bandwidth in the range of 1 KHz to 10 KHz.

6. The power injector (10) of any of Claims 1-5, wherein said piezo motor (150) comprises a Copper Alloy No. 642 and/or is devoid of lead and/or is devoid of piezoelectric crystals.

7. A method for integrating a piezo motor (150) with a power injector (10) used in magnetic resonance imaging, wherein said power injector (10) comprises a syringe plunger driver (14), said method comprising:
tuning a piezo motor (150) to a specific frequency range, wherein said tuning step comprises:
executing a first applying step comprising applying a pressure to said piezo motor (150);
generating at least one signal using a control board, wherein said at least one signal has a selected frequency within said specific frequency range;
executing a second applying step comprising applying said at least one signal to said piezo motor (150);
measuring a vibration frequency of said piezo motor (150);
varying said applied pressure to said piezo motor (150); and
repeating said second applying step, said measuring step, and said varying step until said measured vibration frequency is at a resonant frequency that encompasses said specific frequency range; and
incorporating said tuned piezo motor (150) into said power injector (10) after said tuning step and such that said piezo motor (150) is operatively interconnected with said syringe plunger driver (14).

8. The method of Claim 7, wherein said piezo motor (150) comprises a first stator (156) and a second stator (166), and/or wherein said second applying step comprises applying said at least one signal to one of said first stator (156) or said second stator (166).

9. The method of Claim 8, wherein said varying step comprises one or more of: 1) removing at least one shim from between said first stator (156) and said second stator (166), 2) adding at least one shim between said first stator (156) and said second stator (166), 3) externally compressing opposing ends of a housing portion of said piezo motor (150), and/or 4) changing an amount of compression between said first (156) and second (166) stators.

10. The method of any of Claims 7-9, wherein said at least one signal comprises a plurality of pulses, each pulse having a pulse width, and wherein said control board is operative to adjust said pulse width of each of said plurality of pulses, and wherein said at least one signal optionally has a duty cycle in the range 15% to 40%.

11. The method of any of Claims 7-10, further comprising:
applying at least four signals to said piezo motor (150), each said signal of said at least four signals having a selected frequency within said specific frequency range wherein said selected frequency of each said signal of said at least four signals is optionally the same.

12. The method of Claim 11, wherein said piezo motor (150) comprises a first stator (156) and a second stator (166), said method further comprising:
applying at least two of said at least four signals to said first stator (156) and at least two of said at least four signals to said second stator (166).

13. The method of any of Claims 11-12, wherein each of said at least four signals comprises a plurality of pulses, each pulse having a pulse width, and wherein said control board is operative to adjust said pulse width of each of said at least four signals, and wherein each of said at least four signals optionally has: a duty cycle in the range 15% to 40%; and/or a phase relationship of 90°.

14. The method of any of Claims 7-13, wherein said power injector (10) has an operational frequency range comprising a lowest frequency and a highest frequency, wherein:
a lowest frequency of said specific frequency range for said tuning step is no more than 4 KHz from said lowest frequency of said operational frequency range of said power injector (10), and is further, optionally: no more than 3 KHz from said lowest frequency of said operational frequency range of said power injector (10), no more than 2 KHz from said lowest frequency of said operational frequency range of said power injector (10), or no more than 1 KHz from said lowest frequency of said operational frequency range of said power injector (10), and wherein:
a highest frequency of said specific frequency range for said tuning step is no more than 4 KHz from said highest frequency of said operational frequency range of said power injector (10), and is further, optionally: no more than 3 KHz from said highest frequency of said operational frequency range of said power injector (10), no more than 2 KHz from said highest frequency of said operational frequency range of said power injector (10), or no more than 1 KHz from said highest frequency of said operational frequency range of said power injector (10).

15. The method of Claim 14, wherein said lowest frequency of said specific frequency range for said tuning step is less than said lowest frequency of said operational frequency range of said power injector (10), and wherein said highest frequency of said specific frequency range for said tuning step is greater than said highest frequency of said operational frequency range of said power injector (10).

16. The method of any of Claims 14-15, wherein said selected frequency is a center frequency of said operational frequency range of said power injector (10) and/or wherein said resonant frequency optionally matches said specific frequency range.

## Patentansprüche

1. Leistungsinjektor (10) zum Abbilden von Magnetresonanz, der Folgendes aufweist:
einen Spritzenkolbenantrieb (14);
einen Piezomotor (150), der einen abgestimmten Frequenzbereich aufweist, wobei eine niedrigste Frequenz des abgestimmten Frequenzbereichs für den Piezomotor (150) nicht mehr als 4 kHz von einer niedrigsten Frequenz eines Betriebsfrequenzbereichs des Leistungsinjektors (10) beträgt, wobei eine höchste Frequenz des abgestimmten Frequenzbereichs für den Piezomotor (150) nicht mehr als 4 kHz von einer höchsten Frequenz des Betriebsfrequenzbereichs des Leistungsinjektors (10) beträgt, und wobei der Piezomotor (150) mit dem Spritzenkolbenantrieb (14) zum Bewegen des Spritzenkolbenantriebs (14) in mindestens eine erste Richtung wirkverbunden ist, wobei der Betriebsfrequenzbereich des Leistungsinjektors (10) ein Untersatz des abgestimmten Frequenzbereichs des Piezomotors (150) ist; und
eine Steuertafel (200), die mit dem Piezomotor (150) zum Anlegen mindestens eines Signals einer ausgewählten Frequenz innerhalb des Betriebsfrequenzbereichs an den Piezomotor (150) wirkverbunden ist.

2. Leistungsinjektor (10) nach Anspruch 1, der ferner Folgendes aufweist:
eine Spritze (28), die einen Spritzenkolben (32) aufweist, wobei der Spritzenkolbenantrieb (14) mit dem Spritzenkolben (32) in Wechselwirkung tritt, um den Spritzenkolben (32) zu bewegen, wenn der Spritzenkolbenantrieb (14) durch den Piezomotor (150) bewegt wird.

3. Leistungsinjektor (10) nach einem der Ansprüche 1-2, wobei der Spritzenkolbenantrieb (14) einen Stößel (20) aufweist, der auf einer Gewindeantriebsschraube (18) montiert und entlang davon bewegbar ist, und wobei der Piezomotor (150) optional einen ersten Stator (156), einen zweiten Stator (166), einen ersten Rotor (154) und einen zweiten Rotor (164) aufweist.

4. Leistungsinjektor (10) nach einem der Ansprüche 1-3, wobei die niedrigste Frequenz des abgestimmten Frequenzbereichs für den Piezomotor (150) nicht mehr als 3 kHz von der niedrigsten Frequenz, optional nicht mehr als 2 kHz von der niedrigsten Frequenz und optional nicht mehr als 1 kHz von der niedrigsten Frequenz des Betriebsfrequenzbereichs des Leistungsinjektors (10) beträgt; und wobei die höchste Frequenz des abgestimmten Frequenzbereichs für den Piezomotor (150) nicht mehr als 3 kHz von der höchsten Frequenz, optional nicht mehr als 2 kHz von der höchsten Frequenz und optional nicht mehr als 1 kHz von der höchsten Frequenz des Betriebsfrequenzbereichs des Leistungsinjektors (10) beträgt.

5. Leistungsinjektor (10) nach einem der Ansprüche 1-4, wobei der abgestimmte Frequenzbereich für den Piezomotor (150) eine Bandbreite in dem Bereich von 1 kHz bis 10 kHz hat.

6. Leistungsinjektor (10) nach einem der Ansprüche 1-5, wobei der Piezomotor (150) eine Kupferlegierung Nr. 642 aufweist und/oder frei von Blei und/oder frei von piezoelektrischen Kristallen ist.

7. Verfahren zum Integrieren eines Piezomotors (150) mit einem Leistungsinjektor (10), der beim Abbilden von Magnetresonanz benutzt wird, wobei der Leistungsinjektor (10) einen Spritzenkolbenantrieb (14) aufweist, wobei das Verfahren Folgendes aufweist:
Abstimmen eines Piezomotors (150) auf einen bestimmten Frequenzbereich, wobei der Abstimmungsschritt Folgendes aufweist:
Ausführen eines ersten Anlegungsschritts, der ein Anlegen eines Drucks an den Piezomotor (150) aufweist;
Erzeugen mindestens eines Signals unter Verwendung einer Steuertafel, wobei das mindestens eine Signal eine ausgewählte Frequenz innerhalb des bestimmten Frequenzbereichs hat;
Ausführen eines zweiten Anlegungsschritts, der ein Anlegen des mindestens einen Signals an den Piezomotor (150) aufweist;
Messen einer Schwingungsfrequenz des Piezomotors (150) ;
Variieren des angelegten Drucks an dem Piezomotor (150); und
Wiederholen des zweiten Anlegungsschritts, des Messschritts und des Variierungsschritts, bis die gemessene Schwingungsfrequenz auf einer Resonanzfrequenz ist, die den bestimmten Frequenzbereich umschließt; und
Integrieren des abgestimmten Piezomotors (150) in den Leistungsinjektor (10) nach dem Abstimmungsschritt und derart, dass der Piezomotor (150) mit dem Spritzenkolbenantrieb (14) wirkverbunden ist.

8. Verfahren nach Anspruch 7, wobei der Piezomotor (150) einen ersten Stator (156) und einen zweiten Stator (166) aufweist und/oder wobei der zweite Anlegungsschritt ein Anlegen des mindestens einen Signals an einen des ersten Stators (156) oder des zweiten Stators (166) aufweist.

9. Verfahren nach Anspruch 8, wobei der Variierungsschritt einen oder mehrere von Folgendem umfasst: 1) Entfernen mindestens eines Abstandsstücks von zwischen dem ersten Stator (156) und dem zweiten Stator (166), 2) Hinzufügen mindestens eines Abstandsstücks zwischen dem ersten Stator (156) und dem zweiten Stator (166), 3) externes Komprimieren von gegenüberliegenden Enden eines Gehäuseabschnitts des Piezomotors (150) und/oder 4) Ändern eines Komprimierungsgrads zwischen dem ersten (156) und zweiten (166) Stator.

10. Verfahren nach einem der Ansprüche 7-9, wobei das mindestens eine Signal mehrere Impulse aufweist, wobei jeder Impuls eine Impulsbreite hat und wobei die Steuertafel betrieben wird, um die Impulsbreite jedes der mehreren Impulse anzupassen, und wobei das mindestens eine Signal optional einen Arbeitszyklus in dem Bereich von 15% bis 40% hat.

11. Verfahren nach einem der Ansprüche 7-10, das ferner Folgendes aufweist:
Anlegen von mindestens vier Signalen an den Piezomotor (150), wobei jedes Signal der mindestens vier Signale eine ausgewählte Frequenz innerhalb des bestimmten Frequenzbereichs hat, wobei die ausgewählte Frequenz jedes Signals der mindestens vier Signale optional das gleiche ist.

12. Verfahren nach Anspruch 11, wobei der Piezomotor (150) einen ersten Stator (156) und einen zweiten Stator (166) aufweist, wobei das Verfahren ferner Folgendes aufweist:
Anlegen von mindestens zwei der mindestens vier Signale an den ersten Stator (156) und von mindestens zwei der mindestens vier Signale an den zweiten Stator (166).

13. Verfahren nach einem der Ansprüche 11-12, wobei jedes der mindestens vier Signale mehrere Impulse aufweist, wobei jeder Impuls eine Impulsbreite hat und wobei die Steuertafel betrieben wird, um die Impulsbreite jedes der mindestens vier Signale anzupassen, und wobei jedes der mindestens vier Signale optional Folgendes hat: einen Arbeitszyklus in dem Bereich von 15% bis 40% und/oder ein Phasenverhältnis von 90°.

14. Verfahren nach einem der Ansprüche 7-13, wobei der Leistungsinjektor (10) einen Betriebsfrequenzbereich hat, der eine niedrigste Frequenz und eine höchste Frequenz aufweist, wobei:
eine niedrigste Frequenz des bestimmten Frequenzbereichs für den Abstimmungsschritt nicht mehr als 4 kHz von der niedrigsten Frequenz des Betriebsfrequenzbereichs des Leistungsinjektors (10) beträgt und ferner optional Folgendes beträgt: nicht mehr als 3 kHz von der niedrigsten Frequenz des Betriebsfrequenzbereichs des Leistungsinjektors (10), nicht mehr als 2 kHz von der niedrigsten Frequenz des Betriebsfrequenzbereichs des Leistungsinjektors (10) oder nicht mehr als 1 kHz von der niedrigsten Frequenz des Betriebsfrequenzbereichs des Leistungsinjektors (10), und wobei:
eine höchste Frequenz des bestimmten Frequenzbereichs für den Abstimmungsschritt nicht mehr als 4 kHz von der höchsten Frequenz des Betriebsfrequenzbereichs des Leistungsinjektors (10) beträgt und ferner optional Folgendes beträgt: nicht mehr als 3 kHz von der höchsten Frequenz des Betriebsfrequenzbereichs des Leistungsinjektors (10), nicht mehr als 2 kHz von der höchsten Frequenz des Betriebsfrequenzbereichs des Leistungsinjektors (10) oder nicht mehr als 1 kHz von der höchsten Frequenz des Betriebsfrequenzbereichs des Leistungsinjektors (10) .

15. Verfahren nach Anspruch 14, wobei die niedrigste Frequenz des bestimmten Frequenzbereichs für den Abstimmungsschritt kleiner ist als die niedrigste Frequenz des Betriebsfrequenzbereichs des Leistungsinjektors (10) und wobei die höchste Frequenz des bestimmten Frequenzbereichs für den Abstimmungsschritt größer ist als die höchste Frequenz des Betriebsfrequenzbereichs des Leistungsinjektors (10).

16. Verfahren nach einem der Ansprüche 14-15, wobei die ausgewählte Frequenz eine Mittenfrequenz des Betriebsfrequenzbereichs des Leistungsinjektors (10) ist und/oder wobei die Resonanzfrequenz optional mit dem bestimmten Frequenzbereich übereinstimmt.

## Revendications

1. Injecteur de puissance (10) destiné à une imagerie par résonance magnétique, comprenant :
un élément d'entraînement (14) de piston de seringue ;
un moteur piézoélectrique (150) comprenant une plage de fréquence réglée, dans lequel une fréquence la plus basse de ladite plage de fréquence réglée pour ledit moteur piézoélectrique (150) n'est pas à plus de 4 kHz d'une fréquence la plus basse d'une plage de fréquence opérationnelle dudit injecteur de puissance (10), dans lequel une fréquence la plus élevée de ladite plage de fréquence réglée pour ledit moteur piézoélectrique (150) n'est pas à plus de 4 kHz d'une fréquence la plus élevée de ladite plage de fréquence opérationnelle dudit injecteur de puissance (10), et dans lequel ledit moteur piézoélectrique (150) est interconnecté opérationnellement audit élément d'entraînement (14) de piston de seringue pour déplacer ledit élément d'entraînement (14) de piston de seringue dans au moins une première direction, dans lequel ladite plage de fréquence opérationnelle dudit injecteur de puissance (10) est un sous-ensemble de ladite plage de fréquence réglée dudit moteur piézoélectrique (150) ; et
un panneau de commande (200) interconnecté opérationnellement audit moteur piézoélectrique (150) pour appliquer au moins un signal d'une fréquence sélectionnée dans ladite plage de fréquence opérationnelle audit moteur piézoélectrique (150).

2. Injecteur de puissance (10) selon la revendication 1, comprenant en outre :
une seringue (28) comprenant un piston (32) de seringue, ledit élément d'entraînement (14) de piston de seringue interagissant avec ledit piston (32) de seringue pour déplacer ledit piston (32) de seringue lorsque ledit élément d'entraînement (14) de piston de seringue est déplacé par ledit moteur piézoélectrique (150).

3. Injecteur de puissance (10) selon l'une quelconque des revendications 1 à 2, dans lequel ledit élément d'entraînement (14) de piston de seringue comprend un vérin (20) qui est monté sur une vis d'entraînement filetée (18) et mobile le long de celle-ci et dans lequel ledit moteur piézoélectrique (150) comprend éventuellement un premier stator (156), un second stator (166), un premier rotor (154) et un second rotor (164).

4. Injecteur de puissance (10) selon l'une quelconque des revendications 1 à 3, dans lequel ladite fréquence la plus basse de ladite plage de fréquence réglée pour ledit moteur piézoélectrique (150) n'est pas à plus de 3 kHz de ladite fréquence la plus basse, éventuellement pas à plus de 2 kHz de ladite fréquence la plus basse, et éventuellement pas à plus de 1 kHz de ladite fréquence la plus basse, de ladite plage de fréquence opérationnelle dudit injecteur de puissance (10) ; et dans lequel ladite fréquence la plus élevée de ladite plage de fréquence réglée pour ledit moteur piézoélectrique (150) n'est pas à plus de 3 kHz de ladite fréquence la plus élevée, éventuellement pas à plus de 2 kHz de ladite fréquence la plus élevée, et éventuellement pas à plus de 1 kHz de ladite fréquence la plus élevée, de ladite plage de fréquence opérationnelle dudit injecteur de puissance (10).

5. Injecteur de puissance (10) selon l'une quelconque des revendications 1 à 4, dans lequel ladite plage de fréquence réglée pour ledit moteur piézoélectrique (150) a une bande passante dans la plage de 1 kHz à 10 kHz.

6. Injecteur de puissance (10) selon l'une quelconque des revendications 1 à 5, dans lequel ledit moteur piézoélectrique (150) comprend un alliage de cuivre n° 642 et/ou est dépourvu de plomb et/ou est dépourvu de cristaux piézoélectriques.

7. Procédé permettant d'intégrer un moteur piézoélectrique (150) dans un injecteur de puissance (10) utilisé dans une imagerie par résonance magnétique, dans lequel ledit injecteur de puissance (10) comprend un élément d'entraînement (14) de piston de seringue, ledit procédé comprenant :
le réglage d'un moteur piézoélectrique (150) sur une plage de fréquence spécifique, ladite étape de réglage consistant à :
exécuter une première étape d'application consistant à appliquer une pression audit moteur piézoélectrique (150) ;
générer au moins un signal à l'aide d'un panneau de commande, ledit au moins un signal ayant une fréquence sélectionnée dans ladite plage de fréquence spécifique ;
exécuter une seconde étape d'application consistant à appliquer ledit au moins un signal audit moteur piézoélectrique (150) ;
mesurer une fréquence vibratoire dudit moteur piézoélectrique (150) ;
faire varier ladite pression appliquée audit moteur piézoélectrique (150) ; et
répéter ladite seconde étape d'application, ladite étape de mesure et ladite étape de variation jusqu'à ce que ladite fréquence vibratoire mesurée soit à une fréquence de résonance qui englobe ladite plage de fréquence spécifique ; et
l'intégration dudit moteur piézoélectrique (150) réglé dans ledit injecteur de puissance (10) après ladite étape de réglage et de sorte que ledit moteur piézoélectrique (150) soit interconnecté opérationnellement avec ledit élément d'entraînement (14) de piston de seringue.

8. Procédé selon la revendication 7, dans lequel ledit moteur piézoélectrique (150) comprend un premier stator (156) et un second stator (166), et/ou dans lequel ladite seconde étape d'application consiste à appliquer ledit au moins un signal audit premier stator (156) ou audit second stator (166).

9. Procédé selon la revendication 8, dans lequel ladite étape de variation comprend une ou plusieurs des étapes consistant à : 1) enlever au moins une cale entre ledit premier stator (156) et ledit second stator (166), 2) ajouter au moins une cale entre ledit premier stator (156) et ledit second stator (166), 3) compresser extérieurement des extrémités opposées d'une partie logement dudit moteur piézoélectrique (150), et/ou 4) changer une quantité de compression entre lesdits premier (156) et second (166) stators.

10. Procédé selon l'une quelconque des revendications 7 à 9, dans lequel ledit au moins un signal comprend une pluralité d'impulsions, chaque impulsion ayant une largeur d'impulsion, et dans lequel ledit panneau de commande est efficace pour ajuster ladite largeur d'impulsion de chaque impulsion de ladite pluralité d'impulsions, et dans lequel ledit au moins un signal a éventuellement un cycle opératoire dans la plage de 15 % à 40 %.

11. Procédé selon l'une quelconque des revendications 7 à 10, consistant en outre à :
appliquer au moins quatre signaux audit moteur piézoélectrique (150), chaque dit signal desdits au moins quatre signaux ayant une fréquence sélectionnée dans ladite plage de fréquence spécifique, ladite fréquence sélectionnée de chaque dit signal desdits au moins quatre signaux étant éventuellement identique.

12. Procédé selon la revendication 11, dans lequel ledit moteur piézoélectrique (150) comprend un premier stator (156) et un second stator (166), ledit procédé consistant en outre à :
appliquer au moins deux desdits au moins quatre signaux audit premier stator (156) et au moins deux desdits au moins quatre signaux audit second stator (166).

13. Procédé selon l'une quelconque des revendications 11 à 12, dans lequel chacun desdits au moins quatre signaux comprend une pluralité d'impulsions, chaque impulsion ayant une largeur d'impulsion, et dans lequel ledit panneau de commande est efficace pour ajuster ladite largeur d'impulsion de chacun desdits au moins quatre signaux, et dans lequel chaque signal desdits au moins quatre signaux a éventuellement : un cycle opératoire dans la plage de 15 % à 40 % ; et/ou une relation de phase de 90°.

14. Procédé selon l'une quelconque des revendications 7 à 13, dans lequel ledit injecteur de puissance (10) a une plage de fréquence opérationnelle comprenant une fréquence la plus basse et une fréquence la plus élevée, dans lequel :
une fréquence la plus basse de ladite plage de fréquence spécifique pour ladite étape de réglage n'est pas à plus de 4 kHz de ladite fréquence la plus basse de ladite plage de fréquence opérationnelle dudit injecteur de puissance (10), et est en outre, éventuellement : pas à plus de 3 kHz de ladite fréquence la plus basse de ladite plage de fréquence opérationnelle dudit injecteur de puissance (10), pas à plus de 2 kHz de ladite fréquence la plus basse de ladite plage de fréquence opérationnelle dudit injecteur de puissance (10), ou pas à plus de 1 kHz de ladite fréquence la plus basse de ladite plage de fréquence opérationnelle dudit injecteur de puissance (10), et dans lequel :
une fréquence la plus élevée de ladite plage de fréquence spécifique pour ladite étape de réglage n'est pas à plus de 4 kHz de ladite fréquence la plus élevée de ladite plage de fréquence opérationnelle dudit injecteur de puissance (10), et est en outre, éventuellement : pas à plus de 3 kHz de ladite fréquence la plus élevée de ladite plage de fréquence opérationnelle dudit injecteur de puissance (10), pas à plus de 2 kHz de ladite fréquence la plus élevée de ladite plage de fréquence opérationnelle dudit injecteur de puissance (10), ou pas à plus de 1 kHz de ladite fréquence la plus élevée de ladite plage de fréquence opérationnelle dudit injecteur de puissance (10).

15. Procédé selon la revendication 14, dans lequel ladite fréquence la plus basse de ladite plage de fréquence spécifique pour ladite étape de réglage est inférieure à ladite fréquence la plus basse de ladite plage de fréquence opérationnelle dudit injecteur de puissance (10), et dans lequel ladite fréquence la plus élevée de ladite plage de fréquence spécifique pour ladite étape de réglage est supérieure à ladite fréquence la plus élevée de ladite plage de fréquence opérationnelle dudit injecteur de puissance (10).

16. Procédé selon l'une quelconque des revendications 14 à 15, dans lequel ladite fréquence sélectionnée est une fréquence centrale de ladite plage de fréquence opérationnelle dudit injecteur de puissance (10) et/ou dans lequel ladite fréquence de résonance correspond éventuellement à ladite plage de fréquence spécifique.
